(19) Europäisches Patentamt
European Patent Office
Office européen des brevets

(11) Veröffentlichungsnummer: **0 047 946**
**B1**

(12) # EUROPÄISCHE PATENTSCHRIFT

(45) Veröffentlichungstag der Patentschrift: **15.06.83**

(51) Int. Cl.³: **C 07 C 143/70,** C 07 C 139/00

(21) Anmeldenummer: **81106998.8**

(22) Anmeldetag: **07.09.81**

(54) **Verfahren zur Herstellung von Monohydroperfluoralkansulfonsäurehalogeniden sowie einige spezielle Vertreter dieser Verbindungsklasse.**

(30) Priorität: **12.09.80 DE 3034537**

(43) Veröffentlichungstag der Anmeldung: **24.03.82 Patentblatt 82/12**

(45) Bekanntmachung des Hinweises auf die Patenterteilung: **15.06.83 Patentblatt 83/24**

(84) Benannte Vertragsstaaten: **BE CH DE FR GB IT LI NL**

(56) Entgegenhaltungen: **DE-A-1 967 117**

(73) Patentinhaber: **HOECHST AKTIENGESELLSCHAFT, Postfach 80 03 20, D-6230 Frankfurt/Main 80 (DE)**

(72) Erfinder: **Siegemund, Günter, Dr., Frankfurter Strasse 21, D-6238 Hofheim am Taunus (DE)**
Erfinder: **Schwertfeger, Werner, Dr., Erlenstrasse 8, D-6306 Langgöns (DE)**

**EP 0 047 946 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von Monohydroperfluoralkansulfonsäurehalogeniden sowie einige spezielle Vertreter dieser Verbindungsklasse

Monohydroperfluoralkansulfonsäuren der Formel

$$R_f-CHF-(CF_2)_n-SO_3H$$

mit n = 1 und $R_f$ = Fluor oder Perfluoralkyl sind hauptsächlich durch Addition von Natriumbisulfit an Perfluorolefine [J. Org. Chem. 14, 747 (1949); J. Am. Chem. Soc. 75, 4595 (1953)]:

$$R_f-CF{=}CF_2 \xrightarrow[2.\ H^{\oplus}]{1.\ NaHSO_3} R_f-CHF-CF_2-SO_3H$$

oder durch Hydrolyse von perfluorierten cyclischen Sulfonen [J. Fluorine. Chem. 13, 251 (1979)]:

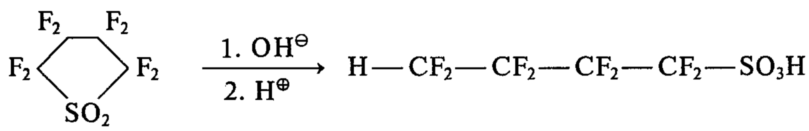


zugänglich.

Aus einer solchen Sulfonsäure wurde in einem Fall mit Hilfe von Phosphorpentachlorid auch bereits das Sulfonsäurechlorid hergestellt [J. Org. Chem. 14, 747 (1949)]:

$$H-CF_2-CF_2-SO_3H \xrightarrow[-POCl_3]{+PCl_5} H-CF_2-CF_2-SO_2Cl$$

Dabei entsteht als Nebenprodukt Phosphoroxichlorid, das von dem Sulfonsäurechlorid nur schlecht abgetrennt werden kann. Eine Ausbeuteangabe fehlt. Das entsprechende Monohydroperfluoralkansulfonsäurefluorid ist bisher nicht bekanntgeworden.

Aufgabe dieser Erfindung war es nun, eine einfachere Methode zur Herstellung von Monohydroperfluoralkansulfonsäurehalogeniden zu finden. Die Methode sollte allgemein anwendbar sein, und die Produkte sollten ohne größeren Aufwand zu reinigen sein.

Diese Aufgabe konnte erfindungsgemäß gelöst werden. Erfindungsgegenstand ist ein Verfahren zur Herstellung von Monohydroperfluoralkansulfonsäurehalogeniden der Formel I

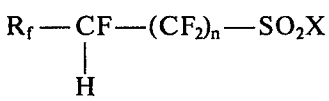
(I)

worin $R_f$ = F oder
Perfluoralkyl mit 1–10, vorzugsweise 1–8, insbesondere 1–3 C-Atomen,
X = Cl oder F, und
n = 1–7, bedeuten,

das dadurch gekennzeichnet ist, daß man

a) Monohydroperfluoralkansulfonsäuren der Formel II

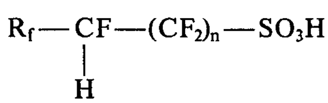
(II)

worin $R_f$ und n die gleiche Bedeutung wie in Formel I besitzen, mit Brenzcatechylphosphortrichlorid

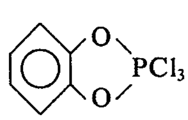

umsetzt zu den Monohydroperfluoralkansulfonsäurechloriden der Formel I mit X = Cl, und daß man

b) diese dann ggf. mit einem Alkalifluorid und/oder Alkalihydrogenfluorid vorzugsweise in einem aprotischen, polaren Lösungsmittel weiter umsetzt zu den Monohydroperfluoralkansulfonsäurefluoriden der Formel I mit X = F.

Dabei war insbesondere das Gelingen der Stufe a) — also das glatte Gelingen der Umsetzung der Monohydroperfluoralkansulfonsäuren der Formel II mit Brenzcatechylphosphortrichlorid zu den Monohydroperfluoralkansulfonsäurechloriden der Formel I mit X = Cl — überraschend, weil das für die Überführung von Carbonsäuren in Carbonsäurechloride bekannte Brenzcatechylphosphortrichlorid [vgl. Chem. Ber. 96, 1387 (1963)] sich hier auch zur Überführung der Sulfonsäuren II in die Sulfonsäurechloride I (mit X = Cl) eignet. Etwa mittels des zur Umwandlung von Carbonsäuren in deren Säurechloride ebenfalls bekannten Thionylchlorids lassen sich nämlich Sulfonsäuren nicht in analoger Weise in die Sulfochloride überführen [J. Org. Chem. 14, 747 (1949)].

Die als Ausgangsverbindungen für das erfindungsgemäße Verfahren eingesetzten Monohydroperfluoralkansulfonsäuren der Formel II sind z. B. nach den eingangs erwähnten bekannten Verfahren erhältlich; d. i. also nach J. Org. Chem. 14, 747 (1949) und J. Am. Chem. Soc. 75, 4595 (1953) aus Perfluorolefinen und Natriumhydrogensulfit, und nach J. Fluorine Chem. 13, 251 (1979) aus perfluorierten cyclischen Sulfonen.

In Stufe a) des Verfahrens wird dann die Sulfonsäure II mit dem Brenzcatechylphosphortrichlorid im allgemeinen etwa im Molverhältnis 1 : 1 umgesetzt. Ein Überschuß an Brenzcatechylphosphortrichlorid ist an sich nicht nötig; ein etwa 5- bis 50%iger Überschuß ist jedoch bevorzugt.

Stufe a) wird im allgemeinen ohne Lösungsmittel durchgeführt; es kann jedoch auch in einem aprotischen inerten Lösungsmittel gearbeitet werden. Wird letzteres (Arbeiten in einem aprotischen inerten Lösungsmittel) getan, so kommen als solche Lösungsmittel beispielsweise in Frage: $CCl_4$, $CHCl_3$, Diglyme oder Tetraglyme etc.

Die Reaktionstemperatur liegt normalerweise zwischen etwa 20 und etwa 180°C, vorzugsweise jedoch zwischen etwa 60 und etwa 140°C.

Es ist für die Durchführung der Stufe a) des erfindungsgemäßen Verfahrens praktisch ohne Belang, in welcher Reihenfolge die Reaktionskomponenten zusammengegeben werden. Von Vorteil ist allerdings, durch gutes Rühren für eine gleichmäßige Durchmischung des Ansatzes zu sorgen.

Nach einer bevorzugten Arbeitsweise wird das Brenzcatechylphosphortrichlorid vorgelegt und über seinen Schmelzpunkt erhitzt. Dann tropft man die Sulfonsäure II zu. Nach dem Ende der Gasentwicklung wird das entstandene Sulfonsäurechlorid der Formel I (mit X = Cl) zweckmäßig durch Destillation abgetrennt.

Aus den Sulfonsäurechloriden der Formel I mit X = Cl lassen sich die Sulfonsäurefluoride der Formel I mit X = F dann nach Stufe b) des erfindungsgemäßen Verfahrens herstellen. Dies erfolgt mittels eines Alkalifluorids und/oder Alkalihydrogenfluorids oder eines Gemisches von mindestens zweien dieser Fluoride, vorzugsweise in einem aprotisch polaren Lösungsmittel.

Für das Gelingen dieser Stufe ist es im Prinzip nicht nötig, mehr als etwa 1 Mol Alkalifluorid und/oder Alkalihydrogenfluorid einzusetzen; ein etwa 5- bis 50%iger Überschuß ist jedoch bevorzugt.

Während in Stufe a) zwar in einem aprotischen inerten Lösungsmittel gearbeitet werden kann, dies dort aber nicht bevorzugt ist, ist in Stufe b) die Verwendung eines solchen Lösungsmittels bevorzugt. Als Lösungsmittel kommen hier in Frage: Nitrile wie Acetonitril oder Butyronitril, Ether wie Diethylenglycoldimethylether oder Tetraethylenglycoldimethylether, Säureamide wie Dimethylformamid oder Dimethylacetamid, Sulfoxide wie Dimethylsulfoxid, Sulfolan etc.

Die Reaktionstemperatur in Stufe b) liegt im allgemeinen zwischen 0 und 160°C, vorzugsweise jedoch zwischen 40 und 120°C.

Auch für Stufe b) ist es — ebenso wie für Stufe a) des Verfahrens — praktisch ohne Belang, in welcher Reihenfolge die Reaktionskomponenten (und das Lösungsmittel) zusammengegeben werden. Eine gleichmäßige Durchmischung des Ansatzes durch gutes Rühren ist auch hier von Vorteil.

Eine bevorzugte Durchführungsweise der Stufe b) besteht darin, das Alkalifluorid und/oder Alkalihydrogenfluorid in dem jeweiligen aprotisch polaren Lösungsmittel vorzulegen und dann mit dem Sulfonsäurechlorid I (mit X = Cl) zu versetzen. Anschließend wird das Gemisch erhitzt. Dabei destilliert das entstehende Sulfofluorid I (mit X = F) ab.

Die erfindungsgemäß hergestellten Monohydroperfluoralkansulfonsäurehalogenide der Formel I sind bis auf die Verbindung $HCF_2-CF_2-SO_2Cl$ (= Verbindung der Formel I mit $R_f = F$, n = 1 und X = Cl) neu.

Die erfindungsgemäß hergestellten Monohydroperfluoralkansulfonsäurehalogenide I sind insbesondere wertvolle Zwischenprodukte in der organischen Fluorchemie. Sie werden mit besonderem Vorteil nach dem Verfahren der gleichzeitig eingereichten Patentanmeldung EP-A-47 945 durch Elektrolyse in einem Elektrolyten aus Fluorsulfonsäure und einem Alkalifluorsulfonat unter Verwendung von Anoden aus glasartigem Kohlenstoff und von Kathoden aus einem üblichen, unter den Elektrolysebedingungen stabilen Material zunächst in die Fluorsulfatoperfluorsulfonsäurehalogenide überführt, aus denen in Gegenwart von Alkalifluoriden als Katalysatoren die entsprechenden Perfluorcarbonylsulfonsäurefluoride erhalten werden:

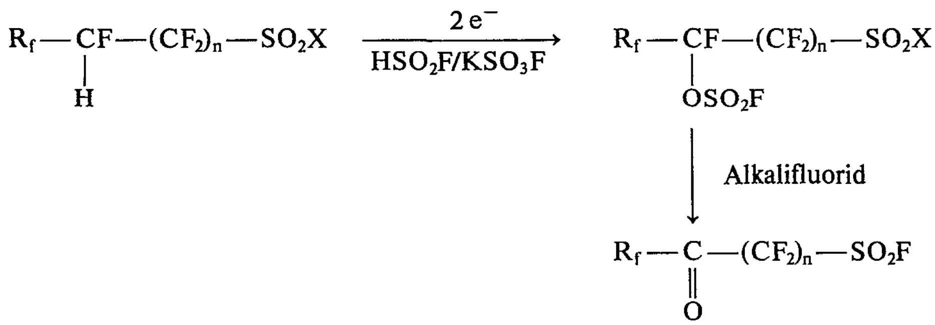


Die erhaltenen Perfluorcarbonylsulfonsäurefluoride können dann ihrerseits auf bekannte Weise z. B. mit Hexafluorpropenepoxid in sulfonsäurefluoridgruppenhaltige Perfluorvinylverbindungen überführt werden,

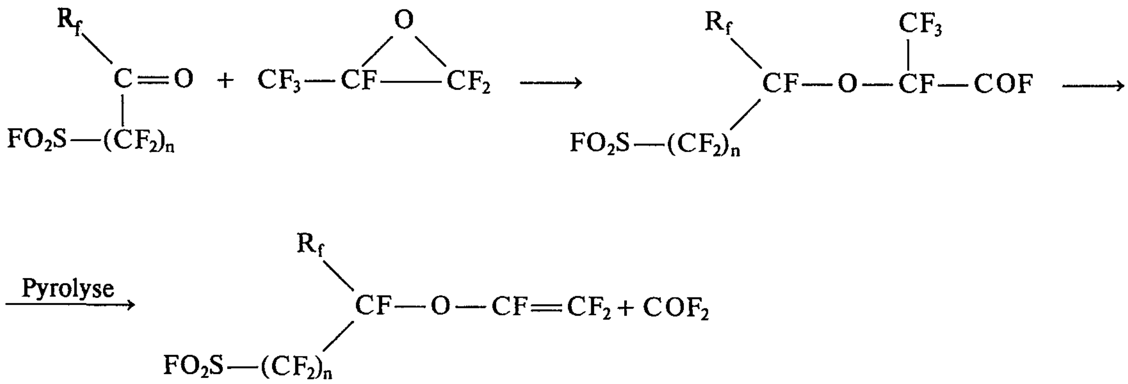


durch deren Homo- oder Copolymerisation (z. B. mit Tetrafluorethylen) mit anschließender Verseifung (der Sulfofluoridgruppen) man zu wertvollen perfluorierten Ionenaustauschern kommt.

Da die vorstehend skizzierte Weiterverarbeitung der Monohydroperfluoralkansulfonsäurehalogenide I mit den Fluoriden (X = F) besser als mit den Chloriden (X = Cl) verläuft, ist es zweckmäßig, dabei von den Fluoriden auszugehen.

Die Erfindung wird nun durch die nachfolgenden Beispiele näher erläutert.

## Beispiel 1

### a) 2-H-Tetrafluorethansulfonsäurechlorid $H-CF_2-CF_2-SO_2Cl$

In einem trockenen Kolben mit Magnetrührer, Rückflußkühler, Tropftrichter und Blasenzähler werden 817 g (3,3 Mol) Brenzcatechylphosphortrichlorid vorgelegt und auf ca. 80°C erwärmt. Dann tropft man 585 g (3,22 Mol) 2-H-Tetrafluorethansulfonsäure zu. Die Reaktion setzt sofort ein und HCl-Gas entweicht. Nachdem die Gasentwicklung beendet ist, wird der Ansatz destilliert. Mit Kp 90—92°C (758 mm) werden 430 g (67%) 2-H-Tetrafluorethansulfonsäurechlorid erhalten.

### b) 2-H-Tetrafluorethansulfonsäurefluorid $H-CF_2-CF_2-SO_2F$

In einem trockenen Kolben mit Rührer, Thermometer, Tropftrichter, Vigreuxkolonne und Kolonnenkopf legt man 400 ml Acetonitril, 145 g (2,5 Mol) Kaliumfluorid und 50 g (0,64 Mol) Kaliumhydrogenfluorid vor. Unter gutem Rühren tropft man 430 g (2,15 Mol) 2-H-Tetrafluorethansulfonsäurechlorid zu. Der Ansatz wird auf 70—80°C Innentemperatur erhitzt. Das entstehende Sulfonsäurefluorid destilliert aus dem Gemisch ab. Die Fraktionierung des Rohproduktes über eine Füllkörperkolonne liefert 358 g (91%) 2-H-Tetrafluorethansulfonsäurefluorid mit Kp 46°C (755 mm).

| | | | | | |
|---|---|---|---|---|---|
| Analyse: | Ber.: | C 13,05 | H 0,55 | F 51,60 | S 17,42 |
| | Gef.: | C 13,0 | H 0,5 | F 51,3 | S 17,4 |

$^1H-NMR$ ($CDCl_3$): 6,20 (ttd, J = 52, 4,5 und ~1,5 Hz)
$^{19}F-NMR$ ($CDCl_3$)*): +44,3 (m, 1F, $-SO_2F$), −114,71 (m, 2F, $-CF_2-S$), −139,95 (dq, J = 52 und 6,5 Hz, 2F, $-CF_2H$)
IR (Gasspektrum): 6,82 μμ (SO)

*) Bei allen $^{19}F$—NMR-Spektren dient $CFCl_3$ als innerer Standard.

Beispiel 2

a) 2-H-Hexafluorpropansulfonsäurechlorid $CF_3-CHF-CF_2-SO_2Cl$

Nach der Arbeitsvorschrift in Beispiel 1 werden 470 g (1,92 Mol) Brenzcatechylphosphortrichlorid und 400 g (1,72 Mol) 2-H-Hexafluorpropansulfonsäure umgesetzt. Es werden 377 (87%) 2-H-Hexafluorpropansulfonsäurechlorid mit Kp 104° C (760 mm) erhalten.

| | | | | | | |
|---|---|---|---|---|---|---|
| Analyse: | Ber.: | C 14,38 | H 0,40 | F 45,50 | Cl 14,15 | S 12,80 |
| | Gef.: | C 14,6 | H 0,5 | F 45,1 | Cl 15,2 | S 13,0 |

| | |
|---|---|
| $^1H-NMR$ ($CDCl_3$): | 5,40 (dm, $^2J_{H,F}$ = 43 Hz) |
| $^{19}F-NMR$ ($CDCl_3$)*): | −73,95 (m, 3F, $CF_3$), −101,6 (δm, $J_{gem}$ = 230 Hz, 1F, $CF_2$), −111,7 ($\delta_m$, $J_{gem}$ = 230 Hz, 1F, $CF_2$), −210,46 (m, 1F, CF) |
| IR (Gasspektrum): | 7,03 μμ (SO) |

b) 2-H-Hexafluorpropansulfonsäurefluorid $CF_3-CHF-CF_2-SO_2F$

Nach der Arbeitsvorschrift in Beispiel 1b setzt man 145 g (2,5 Mol) Kaliumfluorid, 16 g (0,2 Mol) Kaliumhydrogenfluorid und 546 g (2,18 Mol) 2-H-Hexafluorpropansulfonsäurechlorid in 800 ml Butyronitril um. Die Fraktionierung über eine Füllkörperkolonne liefert 312,5 g (61%) 2-H-Hexafluorpropansulfonsäurefluorid mit Kp 63,5° C (760 mm).

| | | | | | |
|---|---|---|---|---|---|
| Analyse: | Ber.: | C 15,39 | H 0,43 | F 56,81 | S 13,70 |
| | Gef.: | C 15,55 | H 0,45 | F 56,65 | S 13,85 |

| | |
|---|---|
| $^1H-NMR$ ($CDCl_3$): | 5,40 (d m, J = 43 Hz) |
| $^{19}F-NMR$ ($CDCl_3$)*): | +41,87 (m, 1F, $SO_2F$), −74,5 (m, 3F, $CF_3$), −106,6 (δm, J = 256 Hz, 1F, $-CF_2-S$), −113,8 (δm, J = 256 Hz, 1F, $-CF_2-S-$), −211,6 (δm, J = 43 Hz, 1F, −CHF) |
| IR (Gasspektrum): | 6,78 μμ (SO) |

**Patentansprüche**

1. Verfahren zur Herstellung von Monohydroperfluoralkansulfonsäurehalogeniden der Formel I

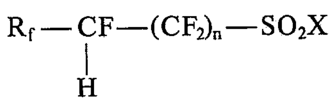

(I)

worin

$R_f$ = F oder Perfluoralkyl mit 1—10, vorzugsweise 1—8, insbesondere 1—3 C-Atomen,
X = Cl oder F und
n = 1—7 bedeuten,

dadurch gekennzeichnet, daß man

a) Monohydroperfluoralkansulfonsäuren der Formel II

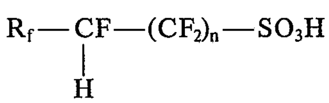

(II)

worin $R_f$ und n die gleiche Bedeutung wie in Formel I besitzen, mit Brenzcatechylphosphortrichlorid umsetzt zu den Monohydroperfluoralkansulfonsäurechloriden der Formel I mit X = Cl, und daß man

b) diese dann gegebenenfalls mit mindestens einem Alkalifluorid und/oder Alkalihydrogenfluorid vorzugsweise in einem aprotischen polaren Lösungsmittel weiter umsetzt zu den Monohydroperfluoralkansulfonsäurefluoriden der Formel I mit X = F.

2. Verfahren nach Anspruch 1, dadurch gekennzeichnet, daß man in Stufe a) pro Mol Monohydroperfluoralkansulfonsäure der Formel II 1 bis 1,5 Mol Brenzcatechylphosphortrichlorid einsetzt.

3. Verfahren nach den Ansprüchen 1 bis 2, dadurch gekennzeichnet, daß man Stufe a) bei Temperaturen zwischen 20 und 180° C, vorzugsweise zwischen 60 und 140° C, durchführt.

4. Verfahren nach den Ansprüchen 1 bis 3, dadurch gekennzeichnet, daß man in Stufe b) pro Mol Monohydroperfluoralkansulfonsäurechlorid der Formel I mit X = Cl 1 bis 1,5 Mol Alkalifluorid und/oder Alkalihydrogenfluorid einsetzt.

5. Verfahren nach den Ansprüchen 1 bis 4, dadurch gekennzeichnet, daß man Stufe b) bei Temperaturen zwischen 0 und 160° C, vorzugsweise zwischen 40 und 120° C, durchführt.

6. Monohydroperfluoralkansulfonsäurehalogenide der Formel I mit Ausnahme der Verbindung $HCF_2-CF_2-SO_2Cl$ ($R_f = F$, n = 1, X = Cl).

## Claims

1. A process for the preparation of monohydroperfluoroalkanesulfonic acid halides of the formula I

$$R_f-\underset{\displaystyle H}{\underset{|}{CF}}-(CF_2)_n-SO_2X \qquad (I)$$

in which $R_f$ denotes F or perfluoroalkyl with 1—10, preferably 1—8 and in particular 1—3, C atoms, X denotes Cl or F and n denotes a number from 1 to 7, characterized in

a) reacting monohydroperfluoroalkanesulfonic acids of the formula II

$$R_f-\underset{\displaystyle H}{\underset{|}{CF}}-(CF_2)_n-SO_3H \qquad (II)$$

in which $R_f$ and n have the same meaning as in formula I, with pyrocatechol-phosphorus trichloride to give the monohydroperfluoroalkanesulfonic acid chlorides of the formula I in which X = Cl, and

b) if desired, then reacting these compounds further with at least one alkali metal fluoride and/or an alkali metal hydrogen fluoride, preferably in an aprotic, polar solvent, to give the monohydroperfluoroalkanesulfonic acid fluorides of the formula I in which X = F.

2. A process as claimed in claim 1, characterized in employing in stage a) 1 to 1.5 moles of pyrocatechol-phosphorus trichloride per mole of monohydroperfluoroalkanesulfonic acid of the formula II.

3. A process as claimed in claim 1 or 2, characterized in carrying out stage a) at temperatures between 20 and 180° C, preferably between 60 and 140° C.

4. A process as claimed in any of claim 1 to 3, characterized in employing in stage b) 1 to 1.5 moles of alkali metal fluoride and/or alkali metal hydrogen fluoride per mole of monohydroperfluoroalkanesulfonic acid chloride of the formula I in which X = Cl.

5. A process as claimed in any of claims 1 to 4, characterized in carrying out stage b) at temperatures between 0 and 160° C, preferably between 40 and 120° C.

6. Monohydroperfluoroalkanesulfonic acid halides of the formula I except the compound $HCF_2-CF_2-SO_2Cl$ ($R_f = F$, n = 1, X = Cl).

## Revendications

1. Un procédé de préparation d'halogénures d'acides monohydroperfluoro-alcane-sulfoniques de formule I

$$R_f-\underset{\displaystyle H}{\underset{|}{CF}}-(CF_2)_n-SO_2X \qquad (I)$$

dans laquelle

- $R_f$ représente le fluor ou un radical perfluoroalkyle en $C_1$ à $C_{10}$, de préférence en $C_1$ à $C_8$ et plus particulièrement en $C_1$ à $C_3$
- X représente Cl ou F et
- n est un nombre de 1 à 7,

procédé caractérisé en ce que

a) on fait réagir des acides monohydroperfluoro-alcanesulfoniques de formule II

$$R_f-\underset{\displaystyle H}{\underset{|}{CF}}-(CF_2)_n-SO_3H \qquad (II)$$

$R_f$ et n ayant les mêmes significations que dans la formule I, avec le trichlorure de pyrocatéchylphosphore pour former les chlorures de sulfonyle correspondants de formule I ci-dessus, X étant le chlore et

b) le cas échéant, on transforme ces chlorures, avec un fluorure et/ou un hydrogénofluorure de métal alcalin, de préférence dans un solvant polaire aprotique, en fluorures de sulfonyle correspondants de formule I, X étant le fluor.

2. Procédé selon la revendication 1, caractérisé en ce que dans l'étape a) on ajoute 1 à 1,5 mole du trichlorure de pyrocatéchylphosphore par mole de l'acide monohydroperfluoro-alcane-sulfonique de formule II.

3. Procédé selon la revendication 1 ou 2, caractérisé en ce que l'on conduit l'étape a) à des températures de 20 à 180° C, de préférence de 60 à 140° C.

4. Procédé selon l'une quelconque des revendications 1 à 3, caractérisé en ce que l'on ajoute dans l'étape b) 1 à 1,5 mole du fluorure et/ou de l'hydrogénofluorure de métal alcalin par mole du chlorure de monohydroperfluoroalcane-sulfonyle de formule I.

5. Procédé selon l'une quelconque des revendications 1 à 4, caractérisé en ce que l'on conduit l'étape b) à une température de 0 à 160° C, de préférence de 40 à 120° C.

6. Les halogénures d'acides monohydroperfluoroalcane-sulfoniques de formule I selon la revendication 1, à l'exclusion du composé $HCF_2-CF_2-SO_2Cl$ ($R_f=F$, $n=1$ et $X=Cl$).